# EUROPÄISCHE PATENTANMELDUNG

(11) **EP 2 202 450 A2**
(43) Veröffentlichungstag der Anmeldung: **30.06.2010**
(21) Anmeldenummer: 10002193.0
(22) Anmeldetag: 11.07.2006
(51) Int. Cl.: F21S 8/00, F21V 14/06

(54) **Operationsleuchte**

(30) Priorität: 02.08.2005 DE 102005036275
(62) Teilanmeldung aus: 06762524.4
(71) Anmelder: Berchtold Holding GmbH, 78532 Tuttlingen (DE)
(72) Erfinder: Scholz, Manfred, 78606 Seitingen-Oberflacht (DE)
(74) Vertreter: Manitz, Finsterwald & Partner GbR

(57) **Zusammenfassung**

Eine Operationsleuchte mit mindestens einer in einem Leuchtenkörper angeordneten Lichtquelle und einem optischen Mittel, um die sichtbare Strahlung der Lichtquelle auf ein Operationsfeld zu richten, weist als Lichtquelle mehrere Leuchtdioden auf.

## Beschreibung

Die vorliegende Erfindung betrifft eine Operationsleuchte mit zumindest einer in einem Leuchtenkörper angeordneten Lichtquelle und einem optischen Mittel, um die sichtbare Strahlung der Lichtquelle auf ein Operationsfeld zu richten.

Derartige Operationsleuchten sind allgemein bekannt, wobei als Lichtquelle Halogenlampen oder Entladungslampen und als optische Mittel Reflektoren eingesetzt werden.

Es ist die Aufgabe der Erfindung, eine Operationsleuchte zu schaffen, die vielseitig verwendbar ist und eine für die Verwendung in einem Operationssaal günstige Bauform aufweist.

Die Lösung dieser Aufgabe erfolgt durch die Merkmale des Anspruchs 1.

Erfindungsgemäß weist die Lichtquelle der Operationsleuchte mehrere Leuchtdioden auf, wodurch sich völlig neue Lösungsansätze für die Operationsfeldbeleuchtung ergeben. Durch die Verwendung von Leuchtdioden als Lichtquelle ist eine flache Bauform der Operationsleuchte möglich, was sowohl in ästhetischer wie auch in strömungstechnischer Hinsicht vorteilhaft ist, da in diesem Fall die Luftströmung einer oberhalb der Operationsstelle befindlichen Zuluftdecke nicht behindert wird. Gleichzeitig ergibt sich durch die gute Dimmbarkeit von Leuchtdioden und durch deren optische Eigenschaften eine Vielzahl von zusätzlichen Anwendungsmöglichkeiten, die in eine Operationsleuchte integriert werden können. Vorteilhafte Ausführungsformen der Erfindung sind in der Beschreibung, der Zeichnung sowie den Unteransprüchen beschrieben.

So ist es vorteilhaft, wenn die Leuchtdioden ringförmig und insbesondere kreisringförmig in dem Leuchtenkörper angeordnet sind. Dies eröffnet die Möglichkeit, im Zentrum des Ringes einerseits Zusatzgeräte oder Zusatzlichtquellen vorzusehen. Andererseits kann der Leuchtenkörper so gestaltet werden, dass der von ihm gebildete Gehäusering in seinem Inneren, d.h. im Inneren des Ringes, von Luft durchströmbar ist. Hierdurch stellt die Operationsleuchte strömungstechnisch gesehen ein geringeres Hindernis dar und es werden bei Verwendung von Zuluftdecken die Strömungswege für laminare Strömung weniger behindert, so dass Partikel und Keime vom Operationsfeld optimiert ferngehalten werden können.

In diesem Zusammenhang ist es auch vorteilhaft, wenn der Leuchtenkörper bzw. der Gehäusering vergleichsweise flach ausgebildet ist, wodurch sich insgesamt ein scheibenförmiger Leuchtenkörper ergibt, der jedoch in seinem Inneren offen ist.

Nach einer weiteren vorteilhaften Ausführungsform weist der Leuchtenkörper eine zentrale Aufnahme für ein Zusatzgerät, insbesondere für eine Zusatzlichtquelle auf. Beispielsweise kann in der zentralen Aufnahme ein spezieller Objektstrahler, eine Kaltlichtquelle oder eine Kamera angeordnet werden oder es können andere Werkzeuge für das Operationsteam darin vorgesehen sein. Eine zentrale Zusatzlichtquelle kann zur Tiefenausleuchtung und zur gezielten Objektbeleuchtung dienen. In diesem Zusammenhang kann es vorteilhaft sein, im Zentrum des Leuchtenkörpers eine Gasentladungslampe oder Halogenlampe vorzusehen, die insbesondere separat, also unabhängig von den Leuchtdioden, schaltbar ist. Auf diese Weise kann beispielsweise eine Tiefenausleuchtung gezielt zugeschaltet werden, wenn der Operateur diese benötigt.

Nach einer weiteren vorteilhaften Ausführungsform sind mehrere Optikmodule vorgesehen, die jeweils mindestens eine Leuchtdiode und einen zugehörigen Reflektor und/oder Kollimator als optisches Mittel umfassen. Derartige Optikmodule lassen sich als sehr kleine Einheiten herstellen, beispielsweise als kostengünstiges Spritzgussteil. Durch den Kollimator wird eine annähernd parallele Lichtstrahlung erzeugt, wobei Abstrahlwinkel in der Größenordnung von 4° möglich sind. Da das von der Leuchtdiode emittierte Licht bereits an der Lichtquelle kollimiert wird, ermöglicht dies eine hocheffiziente Einkopplung des emittierten Lichts in den Strahlengang. Zusätzlich kann ein Kunststoffkollimator, beispielsweise aus hochbrechendem Kunststoff aufgesetzt werden, der mittels Totalreflexion eine weitere Bündelung des Lichtstrahls und somit den gewünschten geringen Abstrahlwinkel bewirkt.

Besonders vorteilhaft ist es, wenn die Optikmodule beweglich, beispielsweise schwenkbar angeordnet sind, da in diesem Fall durch Verschwenken der Optikmodule eine Veränderung der Lichtfeldgröße möglich ist. Auch kann durch eine Bewegung der Optikmodule relativ zu einem gehäusefesten Reflektor oder Kollimator eine Fokussierung erreicht werden. So kann jede Leuchtdiode eine zugeordnete fokussierende Optik aufweisen, wobei beispielsweise durch Verstellung des Relativabstands zwischen Leuchtdiode und fokussierender Optik eine Fokussierung erreicht wird. Besonders vorteilhaft ist es, wenn alle fokussierenden Optiken der Operationsleuchte durch ein Stellelement gemeinsam verstellbar, beispielsweise verschwenkbar sind. Ebenso ist es möglich, durch ein solches Stellelement, beispielsweise einen Stellring, die Optikmodule der Operationsleuchte gemeinsam zu verstellen, insbesondere zu verschwenken.

Nach einer weiteren vorteilhaften Ausführungsform ist ein ringförmiger Reflektor vorgesehen, der beispielsweise an der Unterseite des Leuchtenkörpers angeordnet oder an der Unterseite des Leuchtenkörpers in diesen integriert ist. Durch einen solchen Ringreflektor wird der Durchmesser des Leuchtengehäuses voll genutzt und es werden maximal divergierende Einstrahlwinkel mit optimaler Schlagschattenfreiheit erreicht. Alternativ ist es natürlich auch möglich, die Leuchtdioden direkt konvergierend und divergierend in das Operationsfeld einstrahlen zu lassen.

Nach einer weiteren vorteilhaften Ausführungsform der Erfindung kann das optische Mittel sowohl einen konvergierenden wie auch einen divergierenden Strahlaustritt bewirken. Beispielsweise können zwei Reflektoren vorgesehen sein, von denen einer einen konvergierenden und der andere einen divergierenden Strahlaustritt bewirkt. Alternativ können auch innerhalb eines Reflektors verschiedene konvergierende und divergierende Reflektorabschnitte vorgesehen werden. Auch ist es möglich, konvergierende und divergierende Optikmodule innerhalb der Operationsleuchte vorzusehen, damit sowohl konvergierende wie auch divergierende Lichtstrahlen erzeugt werden, was für eine schattenfreie Beleuchtung der Operationsstelle vorteilhaft ist.

Nach einer weiteren vorteilhaften Ausführungsform der Erfindung sind die Optikmodule so ausgebildet, dass jedes einzelne Optikmodul das Lichtfeld innerhalb eines vorbestimmten Lichtfelddurchmessers vollständig ausleuchtet. Mit anderen Worten wird das Lichtfeld innerhalb des vorbestimmten Lichtfelddurchmessers nicht von verschiedenen Optikmodulen in verschiedenen Segmenten ausgeleuchtet, sondern jedes Optikmodul sorgt für eine vollständige und gleichmäßige Ausleuchtung des Lichtfeldes innerhalb des vorbestimmten Lichtfelddurchmessers, um eine größtmögliche Schattenfreiheit zu gewährleisten.

Nach einer weiteren vorteilhaften Ausführungsform kann zur Kühlung der Lichtquelle innerhalb eines geschlossenen Gehäuses, in dem sich die Lichtquelle befindet, ein Lüfter vorgesehen sein, um eine Zwangsströmung innerhalb des geschlossenen Gehäuses zu bewirken, welche die Wärme von der Lichtquelle abführt. Auf diese Weise wird einerseits Wärme von der Lichtquelle, beispielsweise einer mit einem Kühlkörper versehenen Leuchtdiode, abgeführt. Gleichzeitig ist durch das geschlossene Gehäuse jedoch sichergestellt, dass die Strömung oberhalb des Operationsfeldes nicht negativ beeinträchtigt wird.

Nach einer weiteren vorteilhaften Ausführungsform der Erfindung kann zur Kühlung der Lichtquelle eine Flüssigkeitskühlung vorgesehen sein. Auf diese Weise kann die von der Lichtquelle erzeugte Wärme, die insbesondere für einen störungsfreien Betrieb mit Leuchtdioden abgeführt werden muss, auf effiziente Weise abgeführt werden. Bevorzugt erfolgt die Kühlung der Lichtquelle in diesem Fall mit Kühlwasser.

Nach einem weiteren Aspekt der Erfindung weist die Operationsleuchte zumindest ein Leuchtmittel, beispielsweise eine oder mehrere der bereits erwähnten Leuchtdioden auf, dessen Intensitätsmaximum in einem Bereich von etwa 450 nm liegt, wobei eine Steuereinrichtung vorgesehen ist, mit der dieses zusätzliche Leuchtmittel unabhängig von der Lichtquelle, also beispielsweise den übrigen Leuchtdioden, ansteuerbar ist. Bei dieser Ausführungsform wird die Erkenntnis ausgenutzt, dass Licht auf den Betrachter auch eine physiologische Wirkung hat. Durch Licht bestimmter Wellenlänge im blauen Farbbereich wird ein zweiter Anteil der Sehbahn aktiviert, der als energetischer Anteil den Stoffwechsel und die Hormondrüsen zur rhythmischen Tätigkeit aktiviert. Es ist bekannt, dass durch das Tageslicht die Ausschüttung eines Hormons (Melatonin) gehemmt wird, was zu einer Aktivierung des Kreislaufs dient, da Melatonin den Tag-Nacht-Rhythmus (circadianer Rhythmus) regelt. Durch Photosensoren im menschlichen Auge, die über die Netzhaut gleichmäßig verteilt sind und die ein Bildmuster nicht auflösen können, wird durch das blaue Licht die nächtliche Sekretion von Melatonin bewirkt. Nachdem Operationsteams häufig viele Stunden und oft auch Nachts unter Stress mit höchster Konzentration arbeiten müssen, kann durch eine passende Auswahl des Lichtspektrums der Chirurg so bei seiner Arbeit unterstützt werden, dass er auch während einer schwierigen Notoperation in der Nacht mit maximaler Konzentrationsfähigkeit arbeiten kann. Durch das zusätzliche Leuchtmittel mit Intensitätsmaximum im Bereich von etwa 450 nm lässt sich die Suppression der Melatoninsekretion anregen, so dass die Leistungsfähigkeit des Operationsteams gesteigert wird, auch wenn dieses nachts oder tagsüber über mehrere Stunden operieren muss.

Besonders vorteilhaft ist es, wenn das zusätzliche Leuchtmittel durch die Steuereinrichtung automatisch in Abhängigkeit von bestimmten Parametern zuschaltbar ist. Derartige Parameter können beispielsweise die aktuelle Uhrzeit (Tageszeit) oder die aktuelle Betriebsdauer der Lichtquelle sein, aus der sich auf die bereits verstrichene Operationszeit zurückschließen lässt. Beispielsweise ist es möglich, die Abstrahlung des Leuchtmittels zu steigern, wenn die aktuelle Uhrzeit fortschreitet, d.h. wenn es zunehmend Nacht wird. Auch kann eine Steigerung erfolgen, wenn die Lichtquelle zunehmend eingeschaltet bleibt, d.h. die Operation beispielsweise über mehrere Stunden andauert. Hierdurch kann einerseits eine Ermüdung verhindert und andererseits sogar eine Leistungssteigerung bewirkt werden.

Wie vorstehend bereits erwähnt wurde, kann das Leuchtmittel zur Melatoninunterdrückung entweder ein zusätzliches Leuchtmittel sein. Es ist jedoch auch möglich, das Farbspektrum der Operationsleuchte so zu steuern, dass der gewünschte Wellenlängenbereich von etwa 450 nm mit erhöhter oder zunehmend erhöhter Intensität abgestrahlt wird. Ferner muss das Leuchtmittel zur Melatoninunterdrückung nicht notwendigerweise in Richtung des Operationsfeldes abstrahlen. Vielmehr kann die Abstrahlung auch seitlich oder nach oben, d.h. indirekt erfolgen. Ferner kann das Leuchtmittel auch in einem separaten Leuchtenkörper angeordnet werden.

Nach einem weiteren Aspekt der Erfindung weist die Operationsleuchte eine Steuereinrichtung auf, die mit einem Eingabemittel versehen ist, mit dem eine Referenzwellenlänge oder ein Referenzwellenlängenbereich ausgewählt werden kann, woraufhin die Leuchtdioden der Operationsleuchte und ggf. weitere Leuchtmittel durch die Steuereinrichtung so ansteuerbar sind, dass die von der Operationsleuchte auf das Operationsfeld gerichtete Strahlung überwiegend oder ausschließlich mit der Referenzwellenlänge oder im Referenzwellenlängenbereich abgegeben wird. Bei dieser Ausführungsform weist die erfindungsgemäße Operationsleuchte neben der Möglichkeit, das Operationsfeld zu beleuchten, zusätzlich ein Diagnoselicht (Referenzlicht) auf, um beispielsweise Tumore und unterschiedliche Gewebe einfach und sicher zu lokalisieren. In diesem Zusammenhang sei erwähnt, dass das Referenzlicht bzw. Diagnoselicht nicht notwendigerweise im sichtbaren Bereich liegen muss.

Da Licht in menschliches Gewebe verschieden weit eindringt, kann durch die gezielte Bestrahlung mit schmalbandigem Licht und ggf. kontrastverstärkenden Maßnahmen eine einfache Unterscheidung von unterschiedlichen Geweben erfolgen. Eine Möglichkeit einer solchen Diagnose ist die Fluoreszenzdiagnostik, die auf der selektiven Anreicherung bestimmter Farbstoffe in Tumorzellen beruht, die nach Anregung durch Licht mit bestimmter Wellenlänge sichtbar werden.

Bei der Fluoreszenzdiagnostik wird Körpergewebe gezielt mit beispielsweise blauem Licht geringer Intensität (Referenzlicht) beleuchtet, dessen diffus rückgestreuter Anteil zusammen mit dem entstehenden Fluoreszenzlicht detektiert wird. Die Intensität des blauen Lichts wird so eingestellt, dass Normalgewebe blau erscheint. Die verstärkte Rotfluoreszenz eines zuvor aufgetragenen Wirkstoffes (beispielsweise 5-Aminolävulinsäure) bewirkt jedoch einen Farbumschlag nach rot. Durch Betrachtung der Operationsstelle mit einem optischen Filter, der die blaue Lichtstrahlung ausfiltert, beispielsweise mit Hilfe einer Brille oder einer elektronischen Kamera, welche die Filterung elektronisch bewirkt, können maligne oder krankhafte Zellen erkannt werden, da sich der Wirkstoff in derartigen Zellen eher anreichert als in gesunden Zellen.

Eine weitere Diagnosemöglichkeit ergibt sich durch Bestrahlung mit der Referenzwellenlänge, da Entzündungen in einem Gewebe durch geändertes Absorptions- und Reflektionsverhalten deutlich dunklere Strukturen als das umgebende gesunde Gewebe erzeugen. Mit einem spektral schmalbandigen Licht wird eine eindeutige Diagnose ermöglicht, was bei herkömmlichen Lichtquellen nur mit Hilfe starker Filterung möglich ist. Da farbige Leuchtdioden quasi-monochromatisches Licht mit einer Bandbreite von +/- 20 nm und einer Farbechtheit von nahezu 100 % erzeugen, eignen sich Leuchtdioden besonders gut als Diagnoselicht. Besonders vorteilhaft ist es dabei, wenn die vorstehend genannte Steuereinrichtung ein Schaltmittel aufweist, um zwischen einer Betriebsweise mit Referenzstrahlung und einer Betriebsweise mit tageslichtähnlicher Strahlung umzuschalten, da auf diese Weise besonders schnell zwischen herkömmlichem Operationsbetrieb und Diagnosebetrieb umgeschaltet werden kann.

Nach einer weiteren vorteilhaften Ausführungsform weist die Steuereinrichtung ein Schaltmittel auf, mit dem zwischen einer Referenzstrahlung im Infrarotbereich und einer Referenzstrahlung im Ultraviolettbereich umgeschaltet werden kann. Eine solche Operationsleuchte kann für verschiedene Diagnoseanwendungen eingesetzt werden, wobei zwischen den verschiedenen Anwendungen im Infrarotbereich und im Ultraviolettbereich auf einfache Weise sowie rasch und sicher umgeschaltet werden kann.

Zur Sichtbarmachung des Fluoreszenzspektrums ist es vorteilhaft, wenn in die Operationsleuchte eine elektronische Kamera eingebaut ist, deren Auswertelektronik die gewünschte Filterung bewirkt.

Nachfolgend wird die vorliegende Erfindung anhand einer vorteilhaften Ausführungsform und unter Bezugnahme auf die Zeichnung beschrieben. Es zeigen:
- Fig. 1: eine teilweise geschnittene Seitenansicht einer Operations- leuchte;
- Fig. 2: eine teilweise geschnittene Seitenansicht einer weiteren Aus- führungsform einer Operationsleuchte; und
- Fig. 3: eine Draufsicht auf eine Steuereinrichtung.

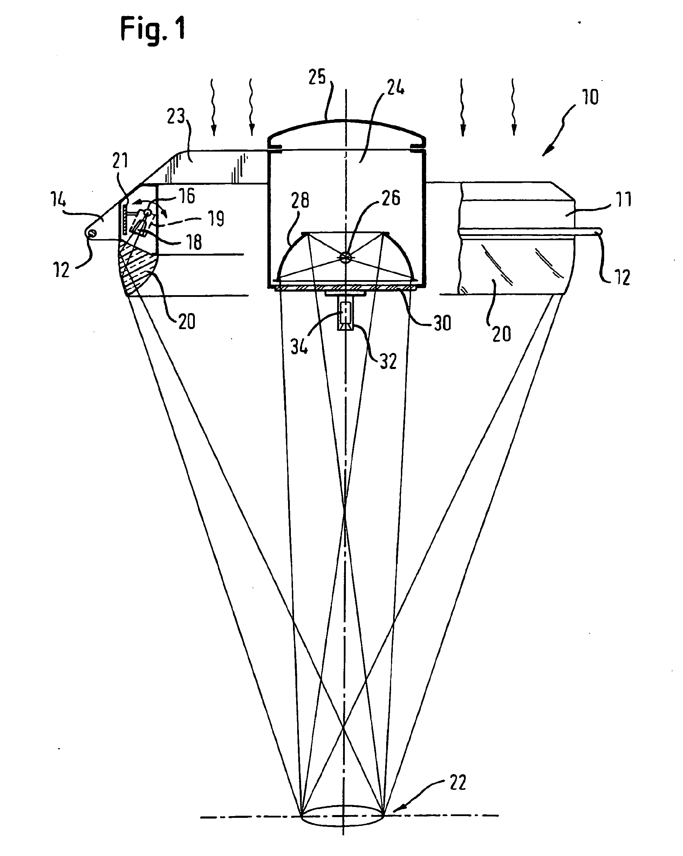

Die in Fig. 1 dargestellte Operationsleuchte weist einen ringförmig ausgebildeten Leuchtenkörper 10 auf, der an seinem Außenumfang von einer Reling 12 umgeben ist, die über Stützelemente 14 an der Außenseite des Leuchtenkörpers 10 befestigt ist. Hierbei ist der Leuchtenkörper 10 als Gehäusering ausgebildet, der beim dargestellten Ausführungsbeispiel kreisringförmig ausgebildet und in seinem Ringinneren von Luft durchströmbar ist, was durch die vertikalen Pfeile in Fig. 1 angedeutet ist. Aufgrund der geringen Höhe des Gehäuseringes ergibt sich insgesamt ein scheibenförmiger Aufbau des Leuchtenkörpers 10.

Im Inneren des Leuchtenkörpers 10 ist eine Lichtquelle in Form einer Vielzahl von Leuchtdioden 16 angeordnet, die über einen Kollimator 18 und einen ringförmigen Reflektor 20 ein Operationsfeld 22 beleuchten. Entsprechend sind die Leuchtdioden 16 ringförmig bzw. kreisringsförmig in dem Leuchtenkörper 10 angeordnet.

Im Inneren des Gehäuseringes 11 ist eine zentrale Aufnahme 24 vorgesehen, die becherartig ausgebildet ist und in ihrem Inneren eine Zusatzlichtquelle 26 in Form einer Entladungslampe aufweist. Die Strahlung der Entladungslampe 26 wird im Inneren der zentralen Aufnahme 24 über einen Parabolreflektor 28 vertikal nach unten gelenkt, um eine Tiefenausleuchtung des Operationsfeldes zu bewirken.

An der Unterseite der zentralen Aufnahme 24 ist eine transparente Abschlussscheibe 30 vorgesehen, in deren Mitte ein Handgriff 32 angeordnet ist, in den eine CCD-Kamera 34 integriert ist. Die Oberseite der zentralen Aufnahme 24 ist mit einem abnehmbaren Gehäusedeckel 25 versehen. Die Operationsleuchte selbst ist über nicht dargestellte Gelenkarme an einem Stativ befestigt. Der Gehäusering 11 ist über drei radial verlaufende Träger 23 mit der zentralen Aufnahme 24 verbunden, die wiederum mit dem nicht dargestellten Gelenkarm in Verbindung steht.

Wie Fig. 1 erkennen lässt, sind bei dieser Ausführungsform die Leuchtdioden 16 und der zugehörige Kollimator 18 in ein gestrichelt dargestelltes Optikmodul 19 integriert, das in Richtung des dargestellten Doppelpfeiles verschwenkt werden kann. Sämtliche Optikmodule 19 sind über einen Stellring 21 gemeinsam verschwenkbar, der sich in Umfangsrichtung innerhalb des Gehäuseringes 11 erstreckt und der mit einer Schrägverzahnung versehen ist, um die Optikmodule entsprechend zu verschwenken, die eine korrespondierende Verzahnung aufweisen. Zum Antrieb des Stellrings 21 ist ein elektrisch betätigbarer Servomotor vorgesehen.

Wie Fig. 1 zeigt, ist der Reflektor 20 ringförmig ausgebildet und an der Unterseite des Gehäuseringes 11 so befestigt, dass er das Innere des Gehäuseringes dicht abschließt. Der Reflektor 20 besteht aus hochbrechendem Kunststoff und reflektiert die einfallende Strahlung durch Totalreflexion an seiner äußeren Mantelfläche. Durch Betätigen des Stellringes 21 kann das Optikmodul 19 verschwenkt werden, wodurch sich der Durchmesser des auf dem Operationsfeld 22 ausgebildeten Lichtfleckes verändert.

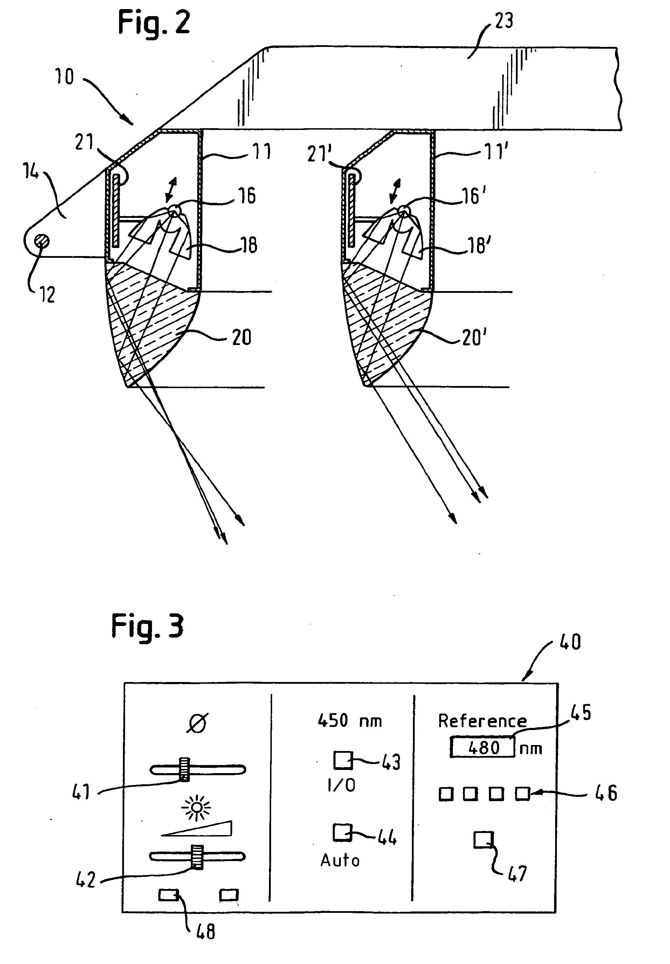

Fig. 2 zeigt eine alternative Ausführungsform einer Operationsleuchte, wobei für gleiche Bauelemente gleiche Bezugszeichen verwendet sind.

Bei der in Fig. 2 dargestellten Operationsleuchte weist der Leuchtenkörper einen ersten Gehäusering 11 auf, der in gleicher Weise wie bei der Ausführungsform von Fig. 1 ausgebildet ist. Zusätzlich ist ein zweiter Gehäusering 11' vorgesehen, der einen geringeren Durchmesser als der erste Gehäusering 11 aufweist, wobei beide Gehäuseringe konzentrisch zueinander angeordnet und an den Trägern 23 befestigt sind. Im Inneren der beiden Gehäuseringe 11 sind wiederum Leuchtdioden 16 und 16' angeordnet, denen jeweils ein Kollimator 18, 18' nachgeordnet ist. Die Unterseite der beiden Gehäuseringe 11 und 11' ist jeweils durch einen ringförmigen Reflektor 20, 20' aus hochbrechendem Kunststoff abgeschlossen.

Die beiden Reflektoren 20 und 20' unterscheiden sich jedoch in der Gestaltung (Krümmung) der äußeren Umfangsfläche, die eine Totalreflexion der einfallenden Strahlung bewirkt. Auf diese Weise verursacht der äußere Ringreflektor 20 einen konvergierenden Strahlenaustritt, während der innere Ringreflektor 20' einen divergierenden Strahlenaustritt bewirkt.

Die jeweiligen Kollimatoren 18 und 18' sind wiederum über Stellringe 21, 21' verstellbar, wobei jedoch bei dieser Ausführungsform keine Schwenkbewegung sondern eine Verstellung des Relativabstands zwischen Leuchtdiode und Kollimator entlang der optischen Achse, d.h. in Richtung des dargestellten Doppelpfeils erfolgt. Bei dieser Ausführungsform sind somit die Leuchtdioden 16, 16' nicht mit den Kollimatoren 18, 18' zu einer Einheit verbunden. Vielmehr handelt es sich bei den Kollimatoren 18, 18' um baulich getrennte Optikmodule, die unabhängig von der Leuchtdiode 16 bewegbar sind.

Besonders vorteilhaft sind Leuchtdioden, bei denen das vom Chip emittierte Licht bereits an der Lichtquelle kollimiert wird, da hierdurch eine hocheffiziente Einkopplung des emittierten Lichts in den Strahlengang ermöglicht wird. Zusätzlich kann ein Kunststoffkollimator nachgeschaltet werden, der mittels Totalreflexion eine weitere Bündelung des Lichtstrahls und somit einen möglichst geringen Abstrahlwinkel in der Größenordnung von 4° bewirkt. Auch können in den Chip oder in die Optikmodule Linsen bzw. Reflektorelemente integriert sein. Darüber hinaus eignen sich besonders Hochleistungs-Leuchtdioden mit integriertem Kühlkörper.

Um eine möglichst variable Farbmischung zu erhalten, können beispielsweise rote, gelbe und blaue Leuchtdioden verwendet werden, wobei die resultierende Farbe über Farbsensoren gezielt geregelt werden kann.

Die in Fig. 3 dargestellte Steuerung 40 enthält eine Stromversorgung (24 V) und ein Interface USB/DMX zum Anschluss einer Steuereinheit. Über das Farbsteuer-Interface werden die verschiedenen LED-Gruppen mit variablen Betriebsspannungen angesteuert, um die gewünschte Farbmischung zu erreichen. Zusätzlich kann es sinnvoll sein, auch weiße Leuchtdioden zu verwenden.

Mit dem oben beschriebenen Farbwechselsystem können alle Farben des sichtbaren Spektrums sowie weißes OP-Licht mit unterschiedlichen Farbtemperaturen erzeugt werden. Für die Ansteuerung von verschiedenen Farbtemperaturen, z.B. 3000 bis 6000 K, ist der Einsatz von amberfarbigen und weißen Leuchtdioden zu bevorzugen.

Die in Fig. 3 dargestellte Steuerung 40 besitzt ein dreigeteiltes Eingabefeld, wobei der linke Teil zur Steuerung des regulären OP-Lichts vorgesehen ist. Mit Hilfe eines Reglers 41 kann der Lichtfelddurchmesser variiert werden, indem der oder die Stellringe 21, 21' über den nicht dargestellten Servomotor betätigt werden. Mit Hilfe des Reglers 42 kann die Helligkeit variiert werden.

Der in Fig. 3 mittlere Teil des Bedienfeldes der Steuerung 40 dient dazu, Strahlung im Wellenlängenbereich von etwa 450 nm mit erhöhter Intensität zuzuschalten, was durch gezieltes Ansteuern der Leuchtdioden 16, 16' oder zusätzlicher Leuchtdioden erfolgen kann. Die Steuerung ist hierbei so ausgelegt, dass das herkömmliche OP-Licht unverändert bleibt, auch wenn die für die Strahlung von 450 nm benötigten Leuchtdioden mit erhöhter Intensität abstrahlen. Die Erhöhung der Strahlung im Bereich von 450 nm (nachfolgend Anregungsstrahlung genannt) kann durch einen Taster 43 dauerhaft zugeschaltet werden. Alternativ ist mit Hilfe des Tasters 44 ein Automatikbetrieb auslösbar, bei dem die Anregungsstrahlung in Abhängigkeit von der aktuellen Uhrzeit und/oder der aktuellen Betriebsdauer der Operationsleuchte mit Hilfe eines vorgegebenen Programms automatisch zugeschaltet wird. Hierbei wird die Anregungsstrahlung mit zunehmender Betriebszeit der Operationsleuchte während einer Operation und mit weiter fortschreitender Tageszeit stetig erhöht. Zu diesem Zweck besitzt die Steuerung 40 eine eingebaute Uhr und eine Zeitmesseinrichtung, die bei Einschalten der Operationsleuchte aktiviert wird, so dass auf die Dauer einer Operation rückgeschlossen werden kann.

Der in Fig. 3 rechte Bereich des Bedienfeldes der Steuerung 40 weist ein Display 45 sowie diverse Eingabemittel 46 auf, mit denen eine Referenzwellenlänge oder .ein Referenzwellenlängenbereich ausgewählt werden kann. In der Darstellung von Fig. 3 ist eine Referenzwellenlänge von 480 nm ausgewählt, was dazu führt, dass nach Freischalten des Referenzlichtes durch Betätigen eines Tasters 47 die Entladungslampe 26 abgeschaltet wird und sämtliche Leuchtdioden 16, 16' so angesteuert werden, dass die von der Operationsleuchte auf das Operationsfeld 22 gerichtete Strahlung ganz überwiegend oder ausschließlich mit der ausgewählten Referenzwellenlänge oder im ausgewählten Referenzwellenlängenbereich abgegeben wird. Auf diese Weise kann ein gewünschtes Diagnoselicht auf einfache Weise eingestellt werden. Durch mehrfaches Betätigen des Tasters 47 wird zwischen einem Betrieb mit Diagnoselicht und einem Betrieb mit herkömmlichem OP-Licht umgeschaltet. Durch einen weiteren Taster 48 ist die Gasentladungslampe 26 getrennt schaltbar.

Die im Handgriff 32 der Operationsleuchte eingebaute Kamera 34 ist mit der Steuerung 40 verbunden, wobei in der Steuerung ein Filtersystem vorgesehen ist, das die erzeugte Referenzstrahlung ausfiltert, so dass lediglich das erzeugte Fluoreszenzspektrum auf einem (nicht dargestellten) Bildschirm wiedergegeben wird.

### Bezugszeichenliste

- 10: Leuchtenkörper
- 11, 11': Gehäusering
- 12: Reling
- 14: Halterung
- 16, 16': Leuchtdioden
- 18, 18': Kollimatoren
- 19: Optikmodul
- 20, 20': Reflektor
- 21, 21': Stellring
- 22: Operationsfeld
- 23: Träger
- 24: zentrale Aufnahme
- 25: Gehäusedeckel
- 26: Gasentladungslampe
- 28: Parabolreflektor
- 30: transparente Scheibe
- 32: Handgriff
- 34: elektronische Kamera
- 40: Steuereinrichtung
- 41,42: Regler
- 43, 44: Taster
- 45: Display
- 46 - 48: Taster

## Patentansprüche

1. Operationsleuchte mit zumindest einer in einem Leuchtenkörper (10) angeordneten Lichtquelle (16) und einem optischen Mittel (18, 18'; 20, 20'), um die sichtbare Strahlung der Lichtquelle auf ein Operationsfeld (22) zu richten, wobei die Lichtquelle mehrere Leuchtdioden (16, 16') aufweist, und
eine Steuereinrichtung (40) vorgesehen ist, die ein Eingabemittel (46) aufweist, mit dem eine Referenzwellenlänge oder ein Referenzwellenlängenbereich ausgewählt werden kann, woraufhin die Leuchtdioden (16, 16') und gegebenenfalls weitere Leuchtmittel (26) der Operationsleuchte durch die Steuereinrichtung (40) so ansteuerbar sind, dass die von der Operationsleuchte auf das Operationsfeld (22) gerichtete Strahlung überwiegend oder ausschließlich mit der Referenzwellenlänge oder im Referenzwellenlängenbereich abgegeben wird.

2. Operationsleuchte nach Anspruch 1,
**dadurch gekennzeichnet,**
**dass** die Steuereinrichtung (40) ein Schaltmittel (47) aufweist, um zwischen einer Betriebsweise mit Referenzstrahlung und einer Betriebsweise mit tageslichtähnlicher Strahlung umzuschalten.

3. Operationsleuchte nach Anspruch 1 oder 2,
**dadurch gekennzeichnet,**
**dass** die Steuereinrichtung (40) ein Schaltmittel aufweist, um zwischen einer Referenzstrahlung im Infrarotbereich und einer Referenzstrahlung im Ultraviolettbereich umzuschalten.

4. Operationsleuchte nach Anspruch 1, 2 oder 3,
**dadurch gekennzeichnet,**
**dass** diese ein elektronisches Kamerasystem (34, 40) aufweist, das zur Aufzeichnung eines Fluoreszenzspektrums ausgebildet ist.

5. Operationsleuchte nach Anspruch 1,
**dadurch gekennzeichnet,**
**dass** die Leuchtdioden (16, 16') ringförmig, insbesondere kreisringförmig, in dem Leuchtenkörper (10) angeordnet sind.

6. Operationsleuchte nach zumindest einem der vorstehenden Ansprüche,
**dadurch gekennzeichnet,**
**dass** der Leuchtenkörper (10) mindestens einen Gehäusering (11, 11') aufweist, der in seinem Ringinneren durchströmbar ist, und in dem die Leuchtdioden (16, 16') angeordnet sind.

7. Operationsleuchte nach zumindest einem der vorstehenden Ansprüche,
**dadurch gekennzeichnet,**
**dass** der Leuchtenkörper (10) scheibenförmig ausgebildet ist.

8. Operationsleuchte nach zumindest einem der vorstehenden Ansprüche,
**dadurch gekennzeichnet,**
**dass** der Leuchtenkörper (10) eine zentrale Aufnahme (24) für ein Zusatzgerät, insbesondere für eine Zusatzlichtquelle (26, 28) aufweist.

9. Operationsleuchte nach zumindest einem der vorstehenden Ansprüche,
**dadurch gekennzeichnet,**
**dass** insbesondere im Zentrum des Leuchtenkörpers (10) eine Gasentladungslampe (26) oder Halogenlampe vorgesehen ist, die insbesondere separat schaltbar ist.

10. Operationsleuchte nach zumindest einem der vorstehenden Ansprüche,
**dadurch gekennzeichnet,**
**dass** mehrere, insbesondere beweglich angeordnete Optikmodule (18, 18', 19) vorgesehen sind, die einen Reflektor und/oder Kollimator (18, 18') als optisches Mittel umfassen, wobei insbesondere der Abstand zwischen einer Leuchtdiode (16) und einem zugehörigen Optikmodul (18, 18') durch ein Stellmittel veränderbar ist.

11. Operationsleuchte nach Anspruch 10,
**dadurch gekennzeichnet,**
**dass** in jedes Optikmodul (19) eine Leuchtdiode (16) integriert ist.

12. Operationsleuchte nach Anspruch 10 oder 11,
**dadurch gekennzeichnet,**
**dass** mehrere Optikmodule (18, 18', 19) durch ein Stellelement, insbesondere einen Stellring (21), gemeinsam verstellbar sind.

13. Operationsleuchte nach zumindest einem der vorstehenden Ansprüche,
**dadurch gekennzeichnet,**
**dass** jede Leuchtdiode (16) als optisches Mittel eine zugeordnete fokussierende Optik aufweist, wobei insbesondere alle fokussierenden Optiken durch ein Stellelement (21) gemeinsam verstellbar, insbesondere verschwenkbar sind.

14. Operationsleuchte nach zumindest einem der vorstehenden Ansprüche,
**dadurch gekennzeichnet,**
**dass** ein ringförmiger Reflektor (20, 20') vorgesehen ist, der insbesondere an der Unterseite des Leuchtenkörpers (10) angeordnet oder dort in den Leuchtenkörper (10) integriert ist.

15. Operationsleuchte nach zumindest einem der vorstehenden Ansprüche,
**dadurch gekennzeichnet,**
**dass** der Reflektor (20, 20') aus Kunststoff besteht und die eingebrachte Strahlung mittels Totalreflexion reflektiert.
